# EUROPEAN PATENT APPLICATION

(11) **EP 1 027 827 A1**
(43) Date of publication of application: **16.08.2000**
(21) Application number: 98921497.8
(22) Date of filing: 28.05.1998
(51) Int. Cl.: A01N 59/00, A01N 37/36

(54) **DISINFECTANT COMPOSITION**

(71) Applicant: Mandalay Inversiones, S.L., 28001 Madrid (ES); Oftrai, S.L., 28760 Tres Cantos (ES)
(72) Inventor: GARCIA NUNEZ, Ma Isabel, E-28001 Madrid (ES); PRIEGO DELGADO, Ma Teresa, E-28760 Tres Cantos (ES)
(74) Representative: Perez Bonal, Bernardo
(86) International application number: ES9800151
(87) International publication number: WO9960859

(57) **Abstract**

The disinfectant composition comprises hydrogen peroxide (3 - 6 %) and lactic acid (1 - 15 %), and, optionally a compound selected from chlorhexidine (0.4 - 5 %) and a laurylsulphate of an alkali metal (0.1 - 3 %). This disinfectant composition, with a broad spectrum of action, can be used in a hospital, veterinary or industrial environment, especially, for disinfecting instruments used in the hospital.

## Description

### FIELD OF THE INVENTION

This invention relates to a broad-spectrum disinfectant composition that comprises hydrogen peroxide and lactic acid, and, optionally, a compound selected from chlorhexidine and alkali metal laurylsulphate. This disinfectant composition can be used in a hospital, veterinary or industrial environment.

### BACKGROUND OF THE INVENTION

Disinfection of instruments used in the hospital constitutes a key point in the effort to control hospital infections. There are various methods and products capable of carrying out this disinfection. A review of the main disinfection methods and products, with an assessment of their applications and risks can be found in "CIRUGÍA. FISIOPATOLOGIA GENERAL. ASPECTOS BASICOS. MANEJO DEL PACIENTE QUIRURGICO" ("SURGERY. GENERAL PHYSIOPATHOLOGY. BASIC ASPECTS. DEALING WITH THE SURGERY PATIENT"). Chapter 39: Disinfection in surgery, by V. Dominguez and R. Herruzo, pages 334-341, Editor: Médica Panamericana; and in the book of talks and communications from the National Meetings on the Advances in Preventative Medicine held by the Virgen de la Arrixaca Hospital in Murcia, on the 14^{th} and 15^{th} of November of 1996, more exactly in the communication entitled "Disinfectants for hospital use, utilisation and risks", R. Herruzo, pages 23-25.

Despite the large variety of disinfectant products available on the market, there are some large gaps in terms of the spectrum of micro-organisms susceptible to these disinfectants. Furthermore, the disinfectants most widely used for disinfecting hospital material present a number of difficulties.

As is well known, 2% glutaraldehyde is the best disinfectant of material used in the hospital and is the choice for high-level disinfection of such material.

Glutaraldehyde is a broad-spectrum disinfectant [active against enterobacteria, Pseudomonas aeruginosa, Mycobacterium tuberculosis, human immuno-deficiency virus (HIV), hepatitis B virus (HBV), hepatitis C virus (HCV), intestinal viruses, etc.] and quick-acting [from 1 to 5 minutes for most micro-organisms, or from 15 to 60 minutes for mycobacteria]. However, glutaraldehyde is less active against atypical mycobacteria, protozoa cysts and it is probably inactive against prions. If the contact time is long enough it can destroy spores independently of the substrate used. It is inactivated relatively little by organic material and acts by eroding the biofilm formed by the micro-organisms. Diluted glutaraldehyde (0.13 % with phenol) is less effective than glutaraldehyde in a 2 % aqueous solution and should not be used in cases when mycobacterial contamination is suspected.

Even so, despite its advantages, use of glutaraldehyde can give rise to diverse problems for the people who handle it as it is allergenic and an irritant either by direct contact with the liquid (provoking irritated dermatitis and exacerbating cases of eczema) or by means of its vapours (provoking rhinitis, conjuntivitis, etc). Irritation depends on the length and degree of exposure [the current maximum exposure permitted is 0.2 parts per million (ppm) in 10 minutes, although this might drop to 0.02 ppm in the future].

Furthermore, glutaraldehyde leads to other effects such as headache, dizziness, nausea, metallic taste and decolourisation or yellowing of the skin. It has also been described as a possible teratogenic and mutagenic agent because of the possibility that it damages deoxyribonucleic acid (DNA), although this observation has not been confirmed *in vivo*. The prevalence of these symptoms is very high in workers exposed to this compound. They have been reported in a range of 33 % - 79 % of the units that use it. In light of this the exposure of the workers to this compound should be limited by using automatic washing and disinfection machines (although these machines sometimes become contaminated, above all with Mycobaterium fortuitum) or by local ventilation systems designed to control the glutaraldehyde vapours and by systematically measuring its atmospheric levels. If exposure cannot be adequately controlled by engineering methods then personal protection should be used (large nitrile-rubber gloves, protective apron, chemical quality eye protection, and breathing apparatus to protect against organic vapours). What is more, it is necessary to supply adequate information to the workers who handle this compound describing the risks involved and to carry out systematic health check-ups.

In light of all this, it is necessary to continue research looking for a better alternative to glutaraldehyde that is just as effective but that does not have the harmful effects produced by this product of reference in disinfection. The choice of new disinfectants, in particular for hospital use, should also take into account the three characteristics demanded of all widely used disinfectants, that is to say, effectiveness, lack of toxicity and price.

The present invention provides an alternative to glutaraldehyde that complies with the characteristics described above.

### DETAILED DESCRIPTION OF THE INVENTION

This invention provides a disinfectant composition that comprises hydrogen peroxide and lactic acid and, optionally, a compound selected from (i) chlorhexidine and (ii) a laurylsulphate of an alkali metal. Additionally, the composition of the invention may contain water and suitable excipients.

Hydrogen peroxide is an oxidising disinfectant of average activity against numerous micro-organisms. The disinfectant composition of the invention can contain between 3 and 6 % by weight hydrogen peroxide with respect to the total weight, and preferably between 5 and 6 % by weight.

Lactic acid increases the efficacy of hydrogen peroxide, reduces the pH of the composition and can be present in the disinfectant composition of the invention in a quantity lying between 1 and 15 % by weight with respect to the total weight, and preferably between 5 and 11 % by weight.

Chlorhexidine is a biguanidine with a powerful anti-microbial activity, although it is not very active against viruses and can affect the lenses of the endoscopes. The disinfectant composition of the invention can contain, in this case, between 0.4 % and 5 % by weight of chlorhexidine with respect to the total weight, preferably between 0.8 and 1.2 %.

Laurylsulphate of an alkali metal is an anionic surfactact, effective as a detergent, and widely used for washing sanitary workers and patients. Laurylsulphate of an alkali metal, preferably sodium laurylsulphate, in the case that the disinfectant composition of the invention contains it, can be present at a quantity lying between 0.1 and 3 % by weight with respect to the total weight, preferably between 0.5 and 1 % by weight.

The disinfectant composition of the invention can also contain a sufficient quantity of water or excipients normally used in the formulation of disinfectant compositions, for example, ferric salts used as food preservatives, and colouring agents.

In a particular embodiment of this invention, the disinfectant composition comprises:

| | |
|---|---|
| Hydrogen peroxide | 3 - 6 % |
| Lactic acid | 1 - 15 % |
| Chlorhexidine | 0.4 - 5 % |
| Water | Sufficient quantity |

where all percentages are expressed by weight with respect to the total weight of the composition.

In another particular embodiment of this invention, the disinfectant composition comprises:

| | |
|---|---|
| Hydrogen peroxide | 3- 6% |
| Lactic acid | 1 - 15 % |
| Laurylsulphate of an alkali metal | 0.1 - 3 % |
| Water | Sufficient quantity |

where all percentages are expressed by weight with respect to the total weight of the composition.

Similarly, when the formation of foam is undesirable, both surfactant agents (chlorhexidine or Laurylsulphate) can be eliminated to give rise, in another particular embodiment of this invention, to a disinfectant composition that comprises:

| | |
|---|---|
| Hydrogen peroxide | 3 - 6 % |
| Lactic acid | 1 - 15 % |
| Water | Sufficient quantity |

where all percentages are expressed by weight with respect to the total weight of the composition.

The disinfectant composition of the invention is useful as a broad-spectrum disinfectant and is especially destined for use in any environment, for example, in hospital, veterinary, industrial, such as food industry, livestock, etc environments, and, in general, in all those environments that require disinfection of pipes, tubing, surfaces and equipment. En particular, the disinfectant composition of the invention turns out to be particularly suitable for disinfecting pipes, surfaces, etc, in a hospital environment as well as material used in the hospital, for example, endoscopes, surgical equipment (forceps, scissors, scalpels, etc.), files for endodontics, etc.

In accordance with this, the invention provides a method for disinfecting pipes and surfaces in the hospital environment which comprises applying a suitable quantity of the disinfectant composition of the invention to the pipes and surfaces to disinfect.

The invention also provides a method for disinfecting instruments used in the hospital that comprises applying a suitable quantity of disinfectant composition of the invention to the instrument used in the hospital to disinfect.

The application of the disinfectant composition of the invention can be carried out by any conventional technique, for example, by means of immersion in a bath that contains the disinfectant composition of the invention, or by spraying, injecting or impregnating, with the help of an appropriate application instrument, the disinfectant composition of the invention onto the pipes, surfaces and instruments to disinfect.

The disinfectant composition of the invention can also be used outside the hospital environment, for example, for disinfecting equipment, instruments, pipes, surfaces, etc., of industrial installations, for example, in the food industry, livestock, etc., in which case, more dilute concentrations than in hospital applications can be used if desired, for example, diluting the disinfectant composition of the invention to give concentrations, 1/2, 1/4, 1/8, etc. that of the concentrated solution, according to the particular case.

In accordance with this, the invention also provides a method for disinfecting equipment, instruments, pipes, surfaces, etc., of industrial installations that comprises applying a suitable quantity of the disinfectant composition of the invention to the equipment, instruments, pipes, surfaces, etc., of the industrial installation to disinfect. The application of the disinfectant composition of the invention can be carried out by any appropriate conventional technique.

Although the disinfectant composition of the invention conserves its activity for, at least, four weeks from its production, it is preferable to use it recently prepared. Even so, the period in which activity is retained can be extended by means of the incorporation of appropriate preserving agents.

The disinfectant composition of the invention acts with a synergistic effect much greater than would be expected by the simple addition of the individual disinfectants present therein. In this sense, it has been surprisingly been found that both the combination of hydrogen peroxide and lactic acid with chlorhexidine or with sodium laurylsulphate produce results as extraordinary as those presented in Examples 3, 4 and 5.

In these tables it can be seen that such combinations increase the bacteriostatic/fungistatic and microbicidal capacity (with respect to the corresponding disinfectants used singly) and reduce the oxidising effect of the hydrogen peroxide without affecting the instruments to disinfect. They are therefore suitable for disinfection of material in the hospital, despite that fact that, as is well known, cases have been described in which hydrogen peroxide in 3 - 6 % aqueous solutions used to disinfect endoscopes and hemodialisers have produced some disorders in the subjects with whom the equipment disinfected with said disinfectant have occurred. On the other hand, chlorhexidine can affect the cement of the lens of endoscopes.

The trials that are described in the following examples have shown that the disinfectant composition of the invention turns out to be very effective for disinfecting material used in the hospital, even complex instruments.

The disinfectant composition of the invention can be easily obtained from mixing components thereof, in suitable quantities, with stirring, until complete homogenisation is achieved.

The following examples constitute particular and illustrative embodiments of this invention and should not be considered as limiting the scope of the invention.

### EXAMPLE 1

### Preparation of a disinfectant composition

A disinfectant composition was prepared that contained:

| | |
|---|---|
| Hydrogen peroxide | 5 % |
| Lactic acid | 5 % |
| chlorhexidine | 0.8 % |
| Water | Sufficient quantity |

The composition was obtained by mixing appropriate quantities of each component and stirring until complete homogenisation was achieved.

### EXAMPLE 2

### Trial of the efficacy of the disinfecting composition

The efficacy of the disinfecting composition described in Example 1 was demonstrated by carrying out the following trials:
a) Study of the bactericidal effect of the composition;
b) Study of the conservation of the activity of the composition; and
c) Study of the deterioration of the instruments.

### 2.1 Study of bactericidal effect

To carry out this trial the following materials were used:
- disinfectant composition from Example 1;
- endodontic files from number 25; and
- micro-organisms:
- 61 micro-organisms obtained from colonisation or infection of diseased people in intensive care units (ICU) distributed in the following manner:
- 29 enterobacteria
- 18 non-fermenting bacteria (NFB), of which 9 were Pseudomonas aeruginosa and the other 9 were Acinetobacter,
- 8 Gram Positive cocci, of which 3 were Enterococcus faecalis and the other 5 Staphylococcus aureus, two of which were metacillin-resistant (MR), and
- 6 yeasts (Candida albicans);
- 1 mycobacterium (Mycobacterium fortuitum ATCC 6841) [10 trials were carried out with this micro-organism]; and
- spores of Bacillus subtilis, obtained from vegative cells transformed into spores by means of ageing and then heating to 80° C, in order to destroy the vegetative cells that remain as such.

The method used to carry out this trial corresponds to the method described by V. Domínguez *et al*. ["Cold sterilization in density practice: An *in vitro* study", L'Igiene Moderna, 1991, 95:654-661]. Essentially this method consists of contaminating number 25 endodontic files (a instrument model with a rough surface) with the micro-organisms and spores mentioned above, a list of which is shown in Table 1. After this contamination, they were brought into contact with the disinfectant composition of Example 1 for 20 minutes, then inactivated by means of the addition of a suitable inhibitor, such as a mixture composed of 6-9 % Tween® 80, 0.5 % sodium bisulphite and 0.5 % sodium thiosulphate.

Next, the germ-carriers are shaken in a culture medium [Tood-Hewitt plus the inhibitor and glass counters] . 0.1 ml of supernatant of these media is used to seed two Müeller-Hinton plates for counting the colony-forming units (CFU). Incubation lasts between 1 and 7 days, depending on the micro-organism.

Some controls were prepared in a similar fashion, with the exception that the germ-carriers were introduced into sterilised distilled water.

The results are expressed as a number of CFU of surviving micro-organisms per millilitre (number micro-org. sup./ml).

In Table 1 the results of the effect of the bactericidal effect of the disinfectant composition of Example 1 are shown.

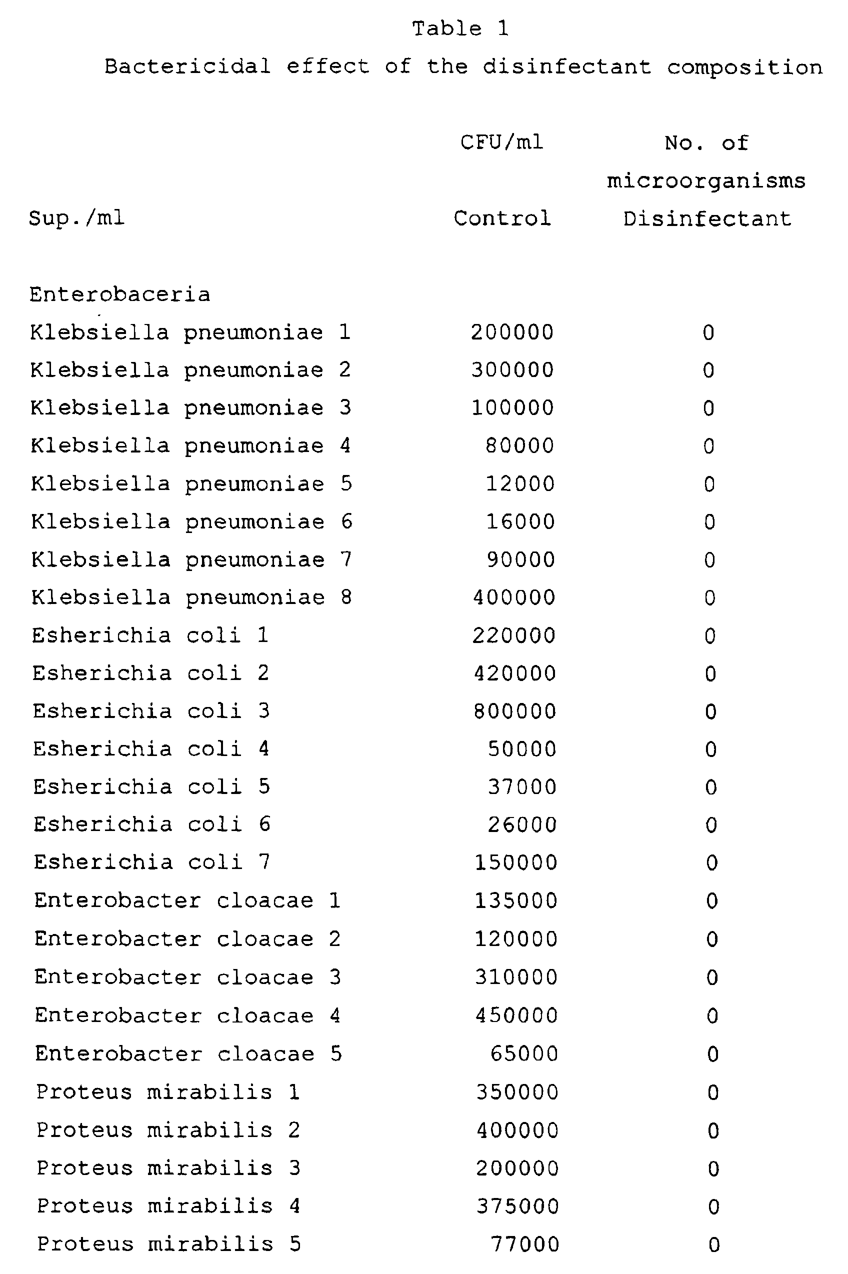

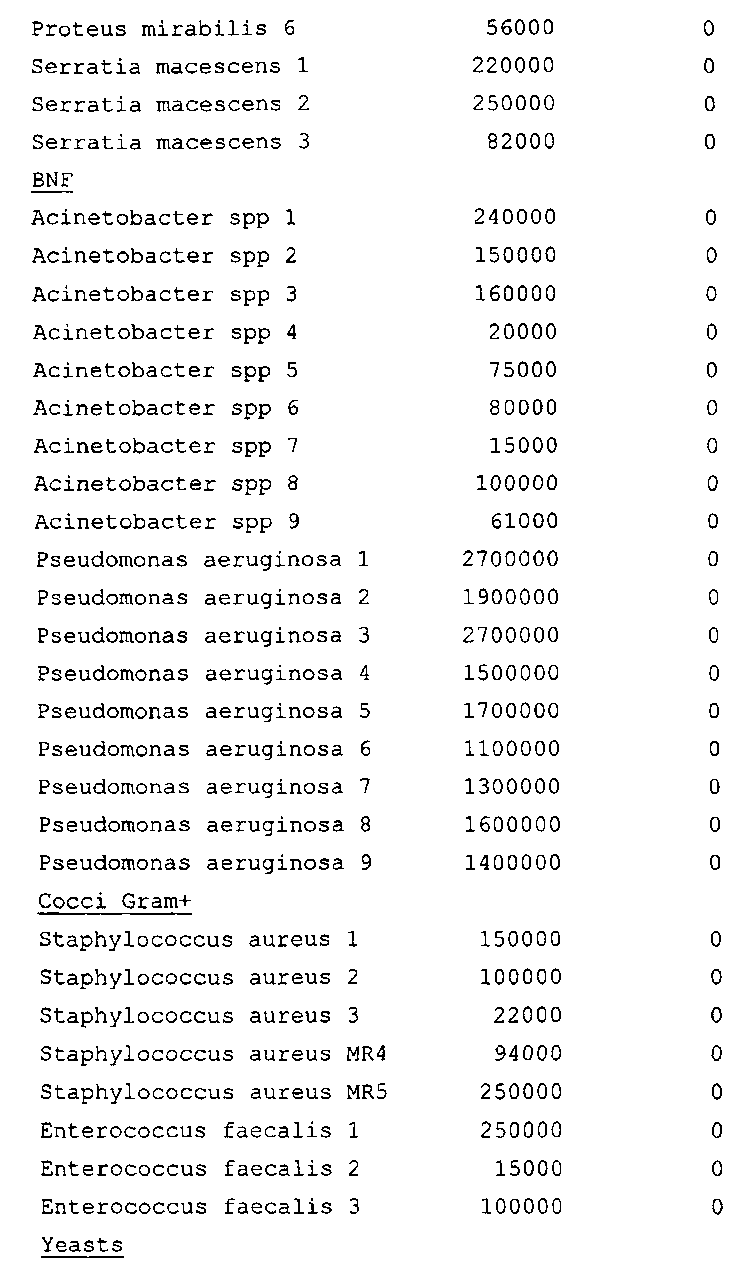

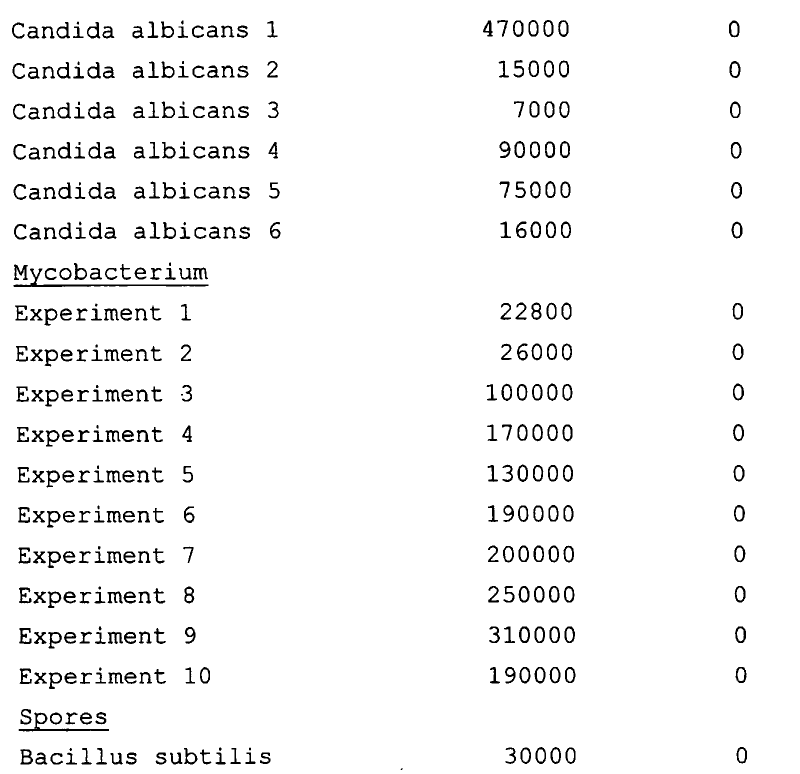

As can be seen in Table 1, the bactericidal effect of the disinfectant composition of Example 1 is complete in the time period studied, against all micro-organisms, including the spores of Bacillus subtilis and Mycobacterium fortuitum (repeated 10 times). Therefore said disinfectant composition shows a similar or superior disinfectant activity compared to glutaraldehyde, persulphate and N-duopropendie [data not shown].

Persulphate [Virkon ®] is a monohydrogen sulphate compound of acidic pH (1.1-2.6), a strong oxidising agent, with activity equivalent to 9.75 % of available chlorine, and so it is effective as a disinfectant although it affects the instruments used in the hospital that are made of poor-quality steel and left submerged for more than 10 minutes. The bactericidal activity of the persulphate is very marked when used at a concentration of 1 % (weight/volume) against non-sporulating bacteria or mycobacteria. It is also active against herpesvirus, reducing more than 4 logarithmic units in 1 minute, but it seems less effective against poliovirus. It is deactivated in the presence of organic material. Its main indications are the disinfection of flexible endoscopes, non-metallic surfaces and glass.

N-duopropenide [NewGer ®] is a mixture of quaternary ammonium iodides in which the iodine is found in bound state, ampholyte, that does not react with acids or bases of low surface tension that show high anti-bacterial efficacy, demonstrated with strains from an extensive multi-centre study in Spanish hospitals, with a very low minimum inhibitory concentration [MIC] and minimum bactericidal concentration [MBC] (geometrical means in the range of 1/512 to 1/65536) . In studies of bactericidal efficacy it appears to be a very quick and effective bactericide, even at low concentrations (0.44 %), but is less effective against mycobacterium. It has a sporicidal effect and is effective against the hepatitis B virus (HBV), the human immuno-deficiency virus (HIV) and poliovirus. Due to the fact that it combines a high anti-microbial efficacy with lack of damage done to the substrate to which it is applied, it can be used as a disinfectant for all types of instruments without problems of toxicity for the people who handle it.

### 2.2 Conservation of the activity

As the critical micro-organisms in the high-level disinfection are the mycobacteria and Pseudomonas aeruginosa, the trial with these micro-organisms has been repeated several times, using a recently prepared disinfectant composition, a preparation after one, two, three or tour weeks of storage at room temperature.

The results are shown in Table 2.

**Table 2**

| Conservation of activity of the disinfectant composition against M. fortuitum (ATTC 6841) and P. aeruginosa | | |
|---|---|---|
| | CFU/ml Control | No. surviving CFU/ml disinfectant |
| | | |

| Mycobacterium fortuitum | | |
|---|---|---|
| Recently prepared | 1800000 | 0 |
| After 7 days | 650000 | 0 |
| After 14 days | 1900000 | 0 |
| After 21 days | 400000 | 0 |
| After 28 days | 800000 | 0 |

| Pseudomonas aeruginosa | | |
|---|---|---|
| Recently prepared | 1500000 | 0 |
| After 7 days | 1900000 | 0 |
| After 14 days | 1000000 | 0 |
| After 21 days | 1600000 | 0 |
| After 28 days | 850000 | 0 |

As can be appreciated in Table 2, the disinfectant composition of Example 1 maintains its activity against the main micro-organisms that are transmitted by endoscopes (mycobacteria and P. aeruginosa), for at least 4 weeks after its preparation. This time can be greater if a suitable stabiliser or preservative is added.

### 2.3 Trial of instrument deterioration

To carry out this trial, scalpels recently removed from their wrapping were introduced into the disinfectant composition of Example 1 for more than 1 month. In no case was deterioration of the instrument observed. This same trial was carried out with other disinfectants leading to corrosion (blackening) of the scalpels after a few minutes (immersion in persulphate), a few hours (immersion in N-duopropenide or glutaraldehyde phenolate 2 %), days (immersion in glutaraldehyde at 2 % or glutaraldehyde phenolate at 1/16), and so the disinfectant composition of Example 1 is shown to be an active but harmless disinfectant for use with hospital material.

On the other hand, some initial studies have been performed to determine the final price of the mixture used, and it is found to lie in the range of other disinfectants used in the hospital environment such as glutaraldehyde at 2 %, N-duopropenide or persulphate.

In view of this, the disinfectant composition of Example 1 is a product that meets the three conditions of efficacy, harmlessness and low price, and so constitutes a suitable substitute for glutaraldehyde at 2 % (which is currently the best disinfectant in use in the hospital) for high-level disinfection.

### EXAMPLE 3

### Demonstration of the synergistic effect between hydrogen peroxide and chlorhexidine

To demonstrate the synergistic effect between hydrogen peroxide and chlorhexidine present in one of the disinfectant compositions of this invention the following trials were carried out:
a) Study of the minimum inhibitory concentration (MIC) on solid medium of compounds by themselves and in combination;
b) Study of the bactericidal effect against Mycobacterium fortuitum; and
c) Study of the oxidising effect during prolonged immersion of the instruments in the composition.

In all the trials the disinfectants used were the following:
A: Aqueous solution of hydrogen peroxide at 5 %
B: Aqueous solution of chlorhexidine at 0.8 %; and
C: Aqueous solution containing 5 % of hydrogen peroxide and 0.8 % of chlorhexidine.

### 3.1 MIC on solid medium

To carry out this trial the following micro-organisms were used in addition to the above mentioned disinfectants:
- Straphylococcus aureus MR, obtained from burn infection of ICU patients suffering from burns;
- Pseudomonas aeruginosa poli R (sensitive to amicacine and ceftazidime), obtained from the burn infection of ICU patients suffering from burns;
- Escherichia coil (resistant to ß-lactam antibiotics, norfloxacine and sulphamethoxazol-trimethoprim), obtained from the urinary infection of paraplegics; and
- Candida spp., obtained from the colonisation of the digestive tract of a ICU patient.

The method used to carry out this trial is described by R. Herruzo *et al*. ["Investigation of different disinfectant deactivating substances to obtain an ideal neutraliser", Rev. Diag. Biol., 1981, 30:117-124], which, briefly, consists of progressively diluting the disinfectants, applying them to the microbial mat, and, after incubation, observing the inhibition halos.

The results obtained are presented in Table 3.

**Table 3**

| MIC on solid medium | | | | |
|---|---|---|---|---|
| Disinfectant | S. aureus | P. aeruginosa | E. coli | Candida |
| A | 1/1024 | 1/256 | 1/64 | 1/64 |
| B | 1/1024 | 1/256 | 1/512 | 1/128 |
| C | 1/4096 | 1/256 | 1/1024 | 1/1024 |

These results show that the combination of hydrogen peroxide with chlorhexidine increases the inhibition capacity as the MIC of both disinfectants is increased indicating a synergistic effect of the two products in combination.

### 3.2. Bactericidal effect against Mycobacterium fortuitum

The bactericidal effect was tested against a micro-organism recognised as being very resistant to disinfection [Mycobacterium fortuitum].

To carry out the study, as well as the above mentioned disinfectants, Mycobacterium fortuitum ATCC 607 was used as the micro-organism.

The method used to carry out this trial is described by V. Dominguez *et al*. ["Cold sterilisation in dentistry practice: An *in vitro* study", L'Igiene Moderna, 1991, 95:654-661], which, essentially, consists of contaminating number 25 endodontic files (an instrument model with a rough surface) with Mycobacterium fortuitum ATCC 607, bringing the contaminated instrument into contact with the disinfectants for different periods of time and deactivating the disinfectant action by added a suitable inhibitor for the disinfectant in question, for example, a mixture composed of 6 - 9 % Tween ® 80, 0.5 % sodium bisulphite and 0.5 % sodium thiosulphate.

Next, the germ-carriers are shaken in a culture medium [Tood-Hewitt plus the inibitor and glass counters]. 0.1 ml of supernatant of these media is used to seed two Müeller-Hinton plates for counting the colony-forming units (CFU). Incubation lasts between 1 and 7 days, depending on the micro-organism. Some controls were prepared in a similar fashion, with the exception that the germ-carriers were introduced into sterilised distilled water.

The results, for each time measured, are expressed as the number of CFU. The results obtained are shown in Table 4.

**Table 4**

| Bactericidal effect on Mycobacterium fortuitum ATCC 607 | | | | |
|---|---|---|---|---|
| | Times (minutes) | | | |
| Disinfectant | 5 | 10 | 15 | 20 |
| A | 376 | 180 | 40 | 2 |
| B | >1000 | >500 | >250 | 25 |
| C | 10 | 0 | 0 | 0 |
| Control: 30,000 CFU | | | | |

These results show that the combination of hydrogen peroxide and chlorhexidine increases the microbicidal capacity and the speed of action even for micro-organisms as resistant as mycobacteria.

### 3.3 Oxidising effect produced by prolonged instrument immersion

To carry out this trial some scalpel blades were used as an example of hospital instruments in addition to the aforementioned disinfectants.

The method used for carry out this trial consists, briefly, in introducing the scalpel blades into the disinfectants to test and evaluating the deterioration of the instruments after a week in the disinfectant at room temperature.

The results obtained are presented in Table 5.

**Table 5**

| Instrument oxidation (scalpels) | | | |
|---|---|---|---|
| Disinfectant | 1 day | 2 days | 1 week |
| A | Yes | Yes | Yes |
| B | No | No | No |
| C | No | No | No |

These results show that the combination of hydrogen peroxide and chlorhexidine reduces the oxidising effect of the hydrogen peroxide, and so does not effect the instruments, while a similar concentration of hydrogen peroxide does have an effect on the instrument model by oxidising it.

### EXAMPLE 4

### Preparation and efficacy of a disinfectant composition that contains sodium laurylsulphate

### 4.1 Preparation

A disinfectant composition was prepared that contained:

| | |
|---|---|
| Hydrogen peroxide | 5 % |
| Lactic acid | 5 % |
| Sodium laurylsulphate | 0.8 % |
| Water | Sufficient quantity |

The composition was obtained by mixing appropriate quantities of each component and stirring until complete homogenisation was attained.

### 4.2 Efficacy study

The efficacy of the germicide composition that contains sodium laurylsulphate was demonstrated by carrying out the trials mentioned in Example 2 [bactericidal effect, conservation of activity and deterioration of material], using the same micro-organisms as those mentioned in said Example 2, with the exception that in the trial for studying the bactericidal effect of the disinfectant composition that contains laurylsulphate the contact time of the contaminated files with said disinfectant composition was 5 minutes instead of 20, obtaining the following results:
a) Bactericidal effect: the bactericidal effect of the disinfectant composition that contains sodium laurylsulphate is complete after 5 minutes against all micro-organisms, including the spores of Bacillus subtilis and Mycobacterium fortuitum (repeated 10 times), and so said disinfectant composition shows a similar or superior activity compared to that obtained with glutaraldehyde persulphate and N-duopropenide [data not shown];
b) Conservation of activity: the disinfectant composition that contains sodium laurylsulphate maintains its activity against the main micro-organisms that are transmitted by means of the endoscopes [mycobacteria and P. aeruginosa], 4 weeks after preparation. This period of time could be increased if a stabiliser or preservative were added; and
c) Trial of material deterioration: no deterioration of material was observed (scalpels recently removed from their wrapping) introduced in the disinfectant composition that contains sodium laurylsulphate for more than 1 month, and so said composition is not only a very active disinfectant but it is also harmless for material used in the hospital.

Initial studies of the final price of this disinfectant composition that contain sodium laurylsulphate indicate that it lies in the range of other disinfectants used in the hospital environment such as 2 % glutaraldehyde, N-duopropenide or persulphate, and so said disinfectant composition complies with the three conditions of efficacy, harmlessness and low price. It therefore constitutes a suitable substitute for 2 % glutaraldehyde for high-level disinfection.

### EXAMPLE 5

### Preparation and efficacy of a disinfectant composition based on hydrogen peroxide and lactic acid

### 5.1 Preparation

A disinfectant composition was prepared that contained 5 % by weight of hydrogen peroxide, 6 % by weight of lactic acid, and sufficient quantity of water.

The composition was obtained by mixing appropriate quantities of each component and stirring until complete homogenisation was attained.

### 5.2 Efficacy study

The efficacy of this germicidal composition was demonstrated by carrying out the trials described in Example 2 [bactericidal effect, conservation of activity and deterioration of material], using the same micro-organisms as those mentioned in the trials described in said Example 2, with the exception that in the trial to study the bactericidal effect of this disinfectant composition the time of contact with the infected files with the disinfectant composition was 10 minutes instead of 20, obtaining the following results:
a) Bactericidal effect: the bactericidal effect of the disinfectant composition based on hydrogen peroxide and lactic acid is complete after 10 minutes against all micro-organisms, including the spores of Bacillus subtilis and the mycobacterium Mycobacterium fortuitum, and so said disinfectant composition shows an activity similar or superior to that shown by glutaraldehyde, persulphate and N-duopropenide [data not shown];
b) Conservation of activity: the disinfectant composition that contains sodium laurylsulphate maintains its activity against the main micro-organisms that are transmitted by means of the endoscopes [mycobacteria and P. aeruginosa], 4 weeks after preparation. This period of time could be increased if a stabiliser or preservative were added; and
c) Trial of material deterioration: no deterioration of material was observed (scalpels recently removed from their wrapping) when introduced in the disinfectant composition that contained sodium laurylsulphate for more than 1 month, and so said composition is not only a very active disinfectant but it is also harmless towards material used in the hospital.

Initial studies of the final price of this disinfectant composition that contains sodium laurylsulphate indicate that it lies in the range of other disinfectants used in the hospital environment such as 2 % glutaraldehyde, N-duopropenide or persulphate, and so said disinfectant composition complies with the three conditions of efficacy, harmlessness and low price, and so it also constitutes a suitable substitute for 2 % glutaraldehyde for high-level disinfection.

### 5.3 Synergistic effect between hydrogen peroxide and lactic acid

To demonstrate the synergistic effect between hydrogen peroxide and lactic acid present in the disinfectant composition of Example 5.1, the trials mentioned in Example 3 were carried out [study of the minimum inhibitory concentration (MIC) on solid medium of the compounds by themselves and mixed together, studies of the bactericidal effect against Mycobacterium fortuitum, and study of the oxidising effect when instruments are immersed for prolonged periods in the disinfectant composition].

In all trials the disinfectants used were the following:
- an aqueous solution of 5 % hydrogen peroxide;
- an aqueous solution of 6 % lactic acid; and
- an aqueous solution containing 5 % hydrogen peroxide and 6 % lactic acid.

The results obtained were as follows:
a) MIC on solid medium: increases more than one dilution in the aqueous solution that contains the hydrogen peroxide and lactic acid with respect to the individual MIC of each one of the products on their own (hydrogen peroxide and lactic acid);
b) Bactericidal effect against Mycobacterium fortuitum: similar results are obtained to those shown in Table 4 (Example 3) (0 micro-organisms surviving after 10 minutes); and
c) Oxidising effect on immersion: deterioration of the metallic material was not observed (scalpel blades) submerged for 1 week in the aqueous solution containing 5 % hydrogen peroxide and 6 % lactic acid while oxidation of the metallic material submerged in the aqueous solution containing 5 % hydrogen peroxide was evident front the first day on.

## Claims

1. A disinfectant composition that comprises hydrogen peroxide and lactic acid.

2. A composition according to claim 1, that comprises between 3 and 6 % by weight of hydrogen peroxide with respect to the total weight.

3. A composition according to claim 1, that comprises between 1 and 15 % by weight of lactic acid with respect to the total weight.

4. A composition according to claim 1, that comprises, furthermore, a compound selected from chlorhexidine and laurylsulphate of an alkali metal.

5. A composition according to claim 4, that comprises between 0.4 and 5 % by weight of chlorhexidine with respect to the total weight.

6. A composition according to claim 4, that comprises between 0.1 and 3 % by weight of laurylsulphate of an alkali metal with respect to the total weight.

7. A composition according to and of the previous claims, that comprises, furthermore, excipients and a sufficient quantity of water.

8. A composition according to claim 1, that comprises:
| | |
|---|---|
| Hydrogen peroxide | 3 - 6 % |
| Lactic acid | 1 - 15 % |
| Water | Sufficient quantity |
where all the percentages are by weight with respect to the total weight of the composition.

9. A composition according to claim 7, that comprises:
| | |
|---|---|
| Hydrogen peroxide | 3 - 6 % |
| Lactic acid | 1 - 15 % |
| Chlorhexidine | 0.4 - 5 % |
| Water | Sufficient quantity |
where all the percentages are by weight with respect to the total weight of the composition.

10. A composition according to claim 7, that comprises:
| | |
|---|---|
| Hydrogen peroxide | 3 - 6 % |
| Lactic acid | 1 - 15% |
| Laurylsulphate of an alkali metal | 0.4 - 5 % |
| Water | Sufficient cuantity |
where all the percentages are by weight with respect to the total weight of the composition.

11. A method for disinfecting instruments used in the hospital that comprises applying a suitable quantity of a disinfectant composition according to any of claims 1 to 10 to the instrument to disinfect.

12. A method for disinfecting pipes and surfaces in a hospital environment that comprises applying a suitable quantity of a disinfecting composition according to any of the claims 1 to 10 to the pipes or surfaces to disinfect.

13. A method for disinfecting equipment, instruments, pipes and surfaces of industrial installations that comprises applying a suitable quantity of a disinfecting composition according to any of the claims 1 to 10 to the pipes or surfaces of the industrial installation to disinfect.

## Amended claims

### Amended claims under Art. 19.1 PCT

A disinfectant composition characterised in that it contains:
| | | |
|---|---|---|
| a) | hydrogen peroxide | 3 - 6 % |
| b) | lactic acid | 5 - 15 % |
| c) | a compound selected from: | |
| | chlorhexidine | 0.4 - 5 %, and |
| | laurylsulphate of alkali metal | 0.1 - 3 % |
| d) | water | sufficient quantity; |
where all percentages are by weight with respect to the total weight of the composition.

A composition according to claim 1, characterised in that it contains between 5 % and 6 % by weight of hydrogen peroxide with respect to the total weight.

A composition according to claim 1, characterised in that it contains between 0.8 and 1.2 % by weight of chlorhexidine with respect to the total weight.

A composition according to claim 1, characterised in that it contains between 0.5 and 1 % by weight of laurylsulphate of an alkali metal with respect to the total weight.

A composition according to claim 1, characterised in that it contains:
| | | |
|---|---|---|
| a) | hydrogen peroxide | 3 - 6 % |
| b) | lactic acid | 5 - 15 % |
| c) | chlorhexidine | 0.4 - 5 %, and |
| d) | water | sufficient quantity; |
where all percentages are by weight with respect to the total weight of the composition.

A composition according to claim 1, characterised in that it contains:
| | | |
|---|---|---|
| a) | hydrogen peroxide | 3 - 6 % |
| b) | lactic acid | 5 - 15 % |
| c) | laurylsulphate of an alkali metal | 0.1 - 3 %, and |
| d) | water | sufficient quantity; |
where all percentages are by weight with respect to the total weight of the composition.

A composition according to claim 1, characterised in that it contains:
| | | |
|---|---|---|
| a) | hydrogen peroxide | 3 - 6 % |
| b) | lactic acid | 5 - 15 % |
| c) | water | sufficient quantity; |
where all percentages are by weight with respect to the total weight of the composition.

A method for disinfecting an instrument used in the hospital that comprises applying a suitable quantity of a disinfectant composition according to any of claims 1 to 6, or 7, to the instrument to disinfect.

A method for disinfecting pipes and surfaces in the hospital environment that comprises applying a suitable quantity of a disinfectant composition according to any of claims 1 to 6, or 7, to the pipes or surfaces to disinfect.

A method for disinfecting equipment, instruments, pipes and surfaces of industrial installations that comprises applying a suitable quantity of a disinfectant composition according to any of claims 1 to 6, or 7, to the equipment, instruments, pipes and surfaces of the industrial installation to disinfect.

Statement under Art. 19.1 PCT
Claims 1 to 13 has been cancelled and replaced with amended claims 1 to 10.

Basis for the amended claims:
Claim 1: original claims 1, 2, 3, 4, 5 and 6 in combination with page 4, lines 2-6 of the specification.
Claim 2: from page 3, line 32 to page 4, line 1 of the specification
Claim 3: page 4, line 12 of the specification
Claim 4: page 4, line 20 of the specification
Claim 5: original claim 9 in combination with page 4, line 6 of the specification.
Claim 6: original claim 10 in combination with page 4, line 6 of the specification.
Claim 7: original claim 8 in combination with page 4, line 6 of the specification.
Claim 8: original claim 11.
Claim 9: original claim 12
Claim 10: original claim 13.
